# EUROPEAN PATENT APPLICATION

(11) **EP 4 397 349 A1**
(43) Date of publication of application: **10.07.2024**
(21) Application number: 24150305.1
(22) Date of filing: 04.01.2024
(51) Int. Cl.: A61M 25/02

(54) **CATHETER ADAPTER WITH INTEGRATED ADHESIVE DISPENSER**

(30) Priority: 04.01.2023 US 202318093208
(71) Applicant: Becton, Dickinson and Company, Franklin Lakes, NJ 07417 (US)
(72) Inventor: SURYAVANSHI, Ajay, 411052 Pune (IN); PRASAD, Shishir, Ramsey, 07446 (US); BHARDWAJ, Himanshu, 201003 Ghaziabad (IN); MARICI, Paul Paia, Piscataway, 08854 (US)
(74) Representative: dompatent von Kreisler Selting Werner - Partnerschaft von Patent- und Rechtsanwälten mbB

(57) **Abstract**

Provided herein is a catheter adapter, including a housing having a proximal end, a distal end, a lumen arranged between and in fluid communication with the distal end and the proximal end, a catheter arranged at the distal end of the catheter adapter and in fluid communication with the lumen, a reservoir, an adhesive received within the reservoir, and one or more fluid flow conduits in fluid communication with the reservoir and configured to direct the adhesive to the skin of a patient on which the catheter adapter is arranged.

## Description

### CROSS-REFERENCE TO RELATED APPLICATION

The present application claims priority to United States Utility Application No. 18/093,208 entitled "Catheter Adapter with Integrated Adhesive Dispenser" filed January 4, 2023, the entire disclosure of which is hereby incorporated by reference in its entirety.

### BACKGROUND OF THE INVENTION

### Field of the Invention

The present invention relates generally to devices and kits for vascular access, in particular devices and kits for vascular access and securing vascular access devices to a patient's skin.

### Description of Related Art

Tissue adhesives for intravascular (IV) catheter securement applications can adhere both the catheter to the insertion site and the catheter hub to the skin, thus reducing catheter movement, migration, and dislodgement. Such tissue adhesive devices work on the principle of placing the adhesive drops at the insertion site and around the catheter hub. Examples of tissue adhesives which can be dispensed through such devices include Dermabond^{®} and Histoacryl^{®}, which form a thin film.

However, due to the macro form of adhesive drops (e.g., a larger drop size) placed for IV catheter securement, the curing time can be long, resulting in a lack of sufficient adhesion strength until curing is complete. This leads to increases in procedure time or inadequate performance while the adhesive cures. The longer waiting time for clinicians is undesirable, and can result in catheter migration and dislodgement. Accordingly, there is a need in the field for improved adhesive delivery for catheter securement.

### SUMMARY OF THE INVENTION

Provided herein is a catheter adapter, including a housing having a proximal end, a distal end, a lumen arranged between and in fluid communication with the distal end and the proximal end, a catheter arranged at the distal end of the catheter adapter and in fluid communication with the lumen, a reservoir, an adhesive received within the reservoir, and one or more fluid flow conduits in fluid communication with the reservoir and configured to direct the adhesive to the skin of a patient on which the catheter adapter is arranged.

In accordance with an embodiment of the present invention, the reservoir is arranged on an upper surface of the catheter adapter housing.

In accordance with an embodiment of the present invention, the reservoir is formed of a compressible material.

In accordance with an embodiment of the present invention, the reservoir is formed of a resilient material.

In accordance with an embodiment of the present invention, the one or more fluid flow conduits includes a first fluid flow conduit in fluid communication with the reservoir and arranged on an upper surface of the catheter adapter housing, the first fluid flow conduit having a proximal end adjacent the reservoir and a distal end adjacent the distal end of the catheter adapter housing, wherein the first fluid flow conduit is configured to direct the adhesive to a location where the catheter pierces the skin of the patient.

In accordance with an embodiment of the present invention, the one or more fluid flow conduits includes a second fluid flow conduit in fluid communication with the reservoir and arranged about a lateral surface of the catheter adapter housing, the second fluid flow conduit having a proximal end arranged on an upper surface of the catheter adapter housing and adjacent the reservoir and a distal end adjacent a bottom surface of the catheter adapter housing. The one or more fluid flow conduits also includes a third fluid flow conduit in fluid communication with the second fluid flow conduit, the third fluid flow conduit extending along an outer surface of the catheter adapter housing parallel to a longitudinal axis defined by the catheter adapter lumen, the third fluid flow conduit having one or more openings configured to direct the adhesive to the skin of the patient.

In accordance with an embodiment of the present invention, the one or more fluid flow conduits are tubular.

In accordance with an embodiment of the present invention, the reservoir and/or the one or more fluid flow conduits are arranged on the catheter adapter housing by a press-fit or a friction fit.

In accordance with an embodiment of the present invention, the reservoir and/or the one or more fluid flow conduits are welded to the catheter adapter housing.

In accordance with an embodiment of the present invention, the reservoir and/or the one or more fluid flow conduits are tack welded or laser welded to the catheter adapter housing.

In accordance with an embodiment of the present invention, the catheter adapter housing includes one or more grooves configured to receive at least a portion of the reservoir and/or the one or more fluid flow conduits.

In accordance with an embodiment of the present invention, the reservoir is spaced from an upper surface of the catheter adapter housing.

In accordance with an embodiment of the present invention, the reservoir is arranged on an upper surface of the housing.

Also provided herein is a kit, including a catheter adapter, including a housing having a proximal end, a distal end, a lumen arranged between and in fluid communication with the distal end and the proximal end, a catheter arranged at the distal end of the catheter adapter and in fluid communication with the lumen, a reservoir, an adhesive received within the reservoir, and one or more fluid flow conduits in fluid communication with the reservoir and configured to direct the adhesive to the skin of a patient on which the catheter adapter is arranged, and a packaging defining an interior, the catheter adapter received within the packaging interior.

In accordance with an embodiment of the present invention, the catheter adapter and/or the packaging are sterilized.

### BRIEF DESCRIPTION OF THE DRAWINGS

**FIG. 1** is a side view of a catheter adapter according to non-limiting embodiments described herein;
**FIG. 2** is an exploded view of a catheter adapter according to non-limiting embodiments described herein;
**FIG. 3** is a front view of a catheter adapter according to non-limiting embodiments described herein;
**FIG. 4** is a bottom view of a portion of a catheter adapter according to non-limiting embodiments described herein;
**FIG. 5** is a side view of a catheter adapter according to non-limiting embodiments described herein;
**FIG. 6** is a perspective view of a catheter adapter according to non-limiting embodiments described herein; and
**FIG. 7** is a side view of a catheter adapter according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

Provided herein are catheter adapters, and kits including the same, which include tissue adhesive reservoirs and conduits for directing adhesives to locations where one or more components of the catheter adapter, such as the catheter, contact or traverse the skin. Such devices allow for more precise delivery of tissue adhesives and a higher level of securement of catheter assemblies to a patient's skin, thus reducing catheter migration and/or dislodgement.

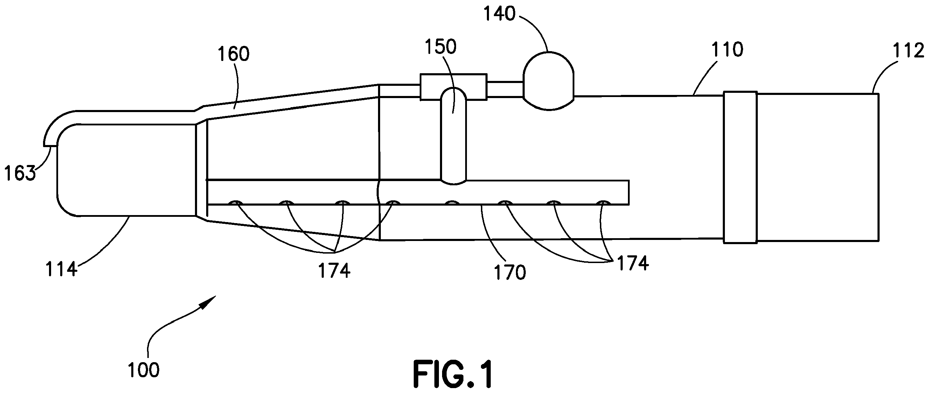

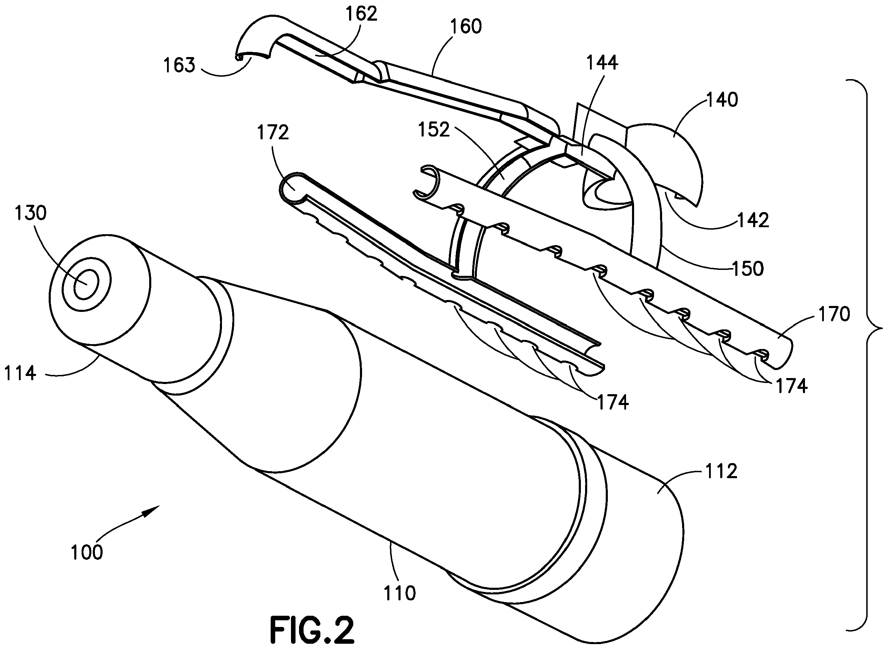

Turning to **FIGS. 1** and **2****,** shown is a non-limiting embodiment of a catheter adapter 100, including a housing 110 having a proximal end (e.g., nearer the care-provider) 112, a distal end (e.g., further from the care-provider) 114, and a sidewall therebetween defining a lumen 130 through which fluid may flow. While not shown in the attached drawings, a catheter adapter 100 may include a catheter for accessing a patient's vasculature. Catheter adapter 100 may include one or more ports, for example at proximal end 112 thereof, or arranged in the sidewall of housing 110, as is known in the art. Any arrangement of tubing may be coupled to catheter adapter 100, for example at such ports, for withdrawing blood from and/or delivering fluids to a patient's vasculature. Housing 110 may be formed of any suitable material, for example one or more plastics. In non-limiting embodiments, catheter adapter housing 110 may taper near distal end 114 thereof.

With continuing reference to **FIGS. 1** and **2****,** catheter adapter 100 may further include, as a co-molded assembly or as a harness that may be friction fit, press-fit, snap-fit, or welded to catheter housing 110, a reservoir 140 and one or more fluid flow conduits 150, 160, 170. Reservoir 140 may define an interior 142 configured to receive a tissue adhesive, that may be used for example to secure catheter adapter 100 to a patient's skin and/or a catheter (not shown) at a site of piercing the patient's skin. Suitable tissue adhesives are known to those of skill in the art and may include cyanoacrylate-containing compositions. In non-limiting embodiments, the tissue adhesive may include an antimicrobial composition. Reservoir 140 may be formed of a material that is compressible, optionally a resiliently compressible material, to allow for a user to apply force and squeeze adhesive out of reservoir interior 142, through conduit 144, for deposition onto the patient's skin.

With reference to **FIG. 2****,** as shown, reservoir 140 may be in fluid communication with one or more fluid flow conduits 150, 160, 170, each defining a fluid flow path 152, 162, 172. In the non-limiting embodiment shown in the accompanying figures, fluid flow conduit 160 may be arranged on an upper surface of catheter adapter housing 110, with a port 163 arranged such that tissue adhesive, flowing through conduit 162 from conduit 144 and reservoir 140, is directed to or near a site where a catheter (not shown) pierces the skin of a patient, thus securing the catheter and reducing and/or eliminating migration and/or dislodgement. Fluid flow conduit(s) 150 may be arranged along an outer surface of the sidewall of housing 110, for example as shown in the accompanying figures, arranged along an outer surface extending from the top surface of housing 110 to adjacent the bottom of housing 110, where fluid flow conduit(s) 150 may join conduit(s) 170.

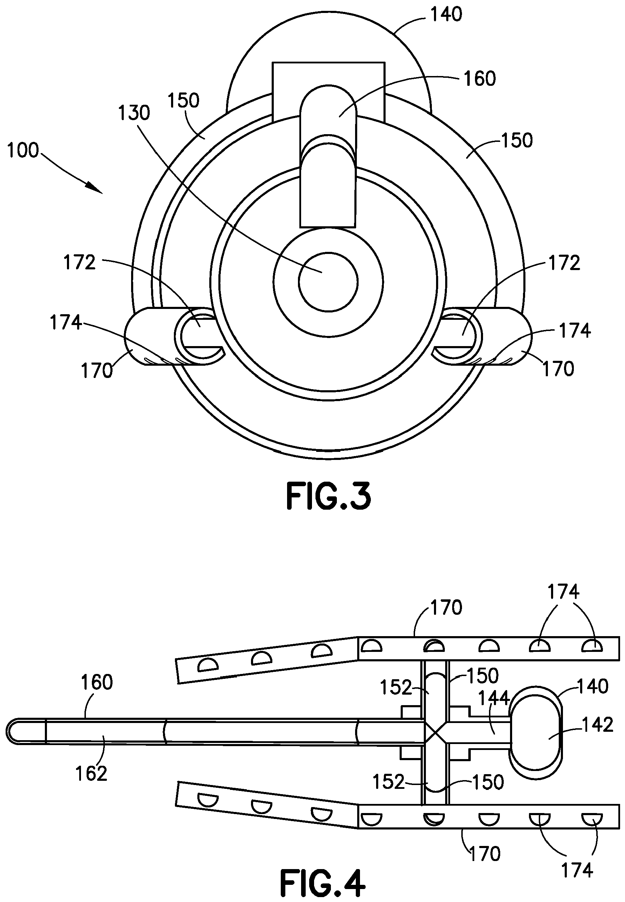

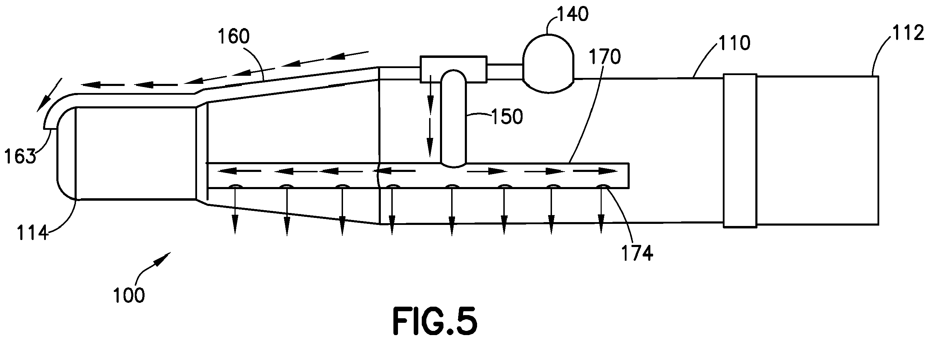

Conduit(s) 170 and path(s) 172 may be in fluid communication with conduit 150 and path 152, which, as noted above, may be in fluid communication with conduit 144 and reservoir 140. Conduit(s) 170 and path(s) 172 may be arranged along a longitudinal axis defined by lumen 130, and may, as shown in **FIG. 3****,** include angled portion(s) near distal end 114 of catheter adapter housing 110. In non-limiting embodiments, for example as shown in the attached figures, conduit(s) 170 may include one or more openings 174 in a bottom surface thereof, configured to direct adhesive flowing through path(s) 172 on a patient's skin where catheter adapter housing 110 is located, thereby securing catheter adapter 100 and reducing and/or eliminating migration and/or dislodgement of a catheter. Exemplary fluid flow paths are shown in **FIG. 5****.**

Turning to **FIG. 3****,** shown is a front view of a non-limiting embodiment of a catheter adapter 100, showing an arrangement of fluid flow conduit 160 relative to lumen 130 and to other components of catheter adapter housing 110. As shown in **FIG. 3****,** fluid flow conduit 170 may be arranged along a taper of catheter adapter housing 110, and thus may be extended radially onward relative to a longitudinal axis defined by lumen 130. Turning to **FIG. 4****,** the arrangement of reservoir 140 and conduits 150, 160, and 170, including opening(s) 174, is shown. As described above, conduit(s) 170 may be angled at a distal end 114 of catheter adapter housing 110 thereof. As also described above, an assembly including reservoir 140 and conduits 150, 160, and 170 may be integral to catheter adapter housing 110, and/or may be fitted and/or welded to catheter adapted housing 110. Such techniques, such as friction fit, press-fit, snap-fit, tack welding, and laser welding, are known to those of skill in the art. In non-limiting embodiments, reservoir 140 and/or conduits 150, 160, and/or 170 may be received within one or more grooves in catheter adapter housing 110.

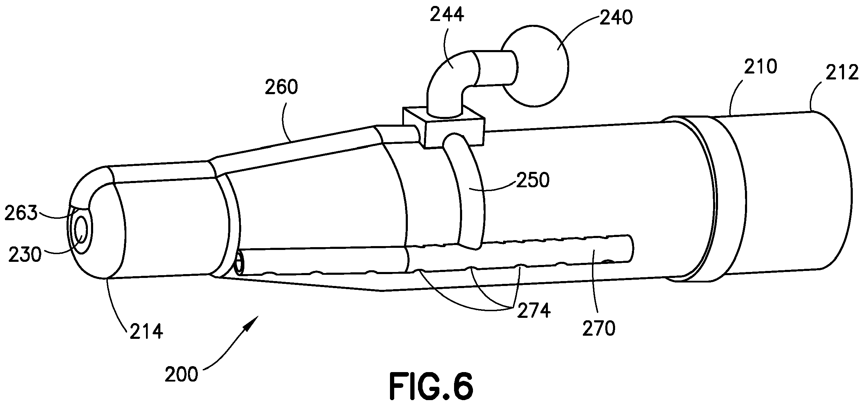

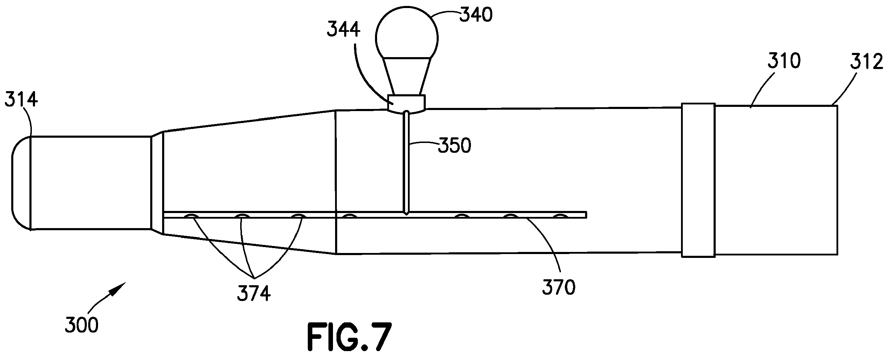

Turning to **FIGS. 6** and **7****,** shown are non-limiting embodiments of alternative arrangements of catheter adapter 200, 300. As described above, catheter adapter 200, 300 may include a housing 210, 310 having a proximal end 212, 312, a distal end 214, 314, and a sidewall therebetween defining a lumen 230 through which fluid may flow. While not shown in the attached drawings, catheter adapter 200, 300 may include a catheter for accessing a patient's vasculature. Catheter adapter 200, 300 may include one or more ports, for example at proximal end 212, 312 thereof, or arranged in the sidewall of housing 210, 310, as is known in the art. Any arrangement of tubing may be coupled to catheter adapter 200, 300, for example at such ports, for withdrawing blood from and/or delivering fluids to a patient's vasculature. Housing 210, 310 may be formed of any suitable material, for example one or more plastics. In non-limiting embodiments, catheter adapter housing 210, 310 may taper near distal end 214, 314 thereof.

The embodiments of **FIGS. 6** and **7** show alternative arrangements of reservoirs 240, 340. In both, reservoir 240, 340 is arranged such that it is spaced from catheter adapter housing 210, 310. In **FIG. 6****,** conduit 244 is curved, placing reservoir 240 in fluid communication with conduits 250, 260, and ultimately, 270. In **FIG. 7****,** reservoir 340 is arranged directly superior to conduit 344 and conduit 350, allowing adhesive to flow to conduit(s) 370, for example passively by gravity flow and/ or under the application of the squeezing force on the reservoir 340 which may be made of a compressible material, optionally a resiliently compressible material, and through opening(s) 374 to secure catheter adapter 300 to a patient's skin.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A catheter adapter, comprising:
a housing having a proximal end, a distal end, a lumen arranged between and in fluid communication with the distal end and the proximal end;
a catheter arranged at the distal end of the catheter adapter and in fluid communication with the lumen;
a reservoir;
an adhesive received within the reservoir; and
one or more fluid flow conduits in fluid communication with the reservoir and configured to direct the adhesive to the skin of a patient on which the catheter adapter is arranged.

2. The catheter adapter of claim 1, wherein the reservoir is arranged on an upper surface of the catheter adapter housing.

3. The catheter adapter of claim 1, wherein the reservoir is formed of a compressible material.

4. The catheter adapter of claim 1, wherein the reservoir is formed of a resilient material.

5. The catheter adapter of claim 1, wherein the one or more fluid flow conduits comprise:
a first fluid flow conduit in fluid communication with the reservoir and arranged on an upper surface of the catheter adapter housing, the first fluid flow conduit having a proximal end adjacent the reservoir and a distal end adjacent the distal end of the catheter adapter housing, wherein the first fluid flow conduit is configured to direct the adhesive to a location where the catheter pierces the skin of the patient.

6. The catheter adapter of claim 1, wherein the one or more fluid flow conduits comprise:
a second fluid flow conduit in fluid communication with the reservoir and arranged about a lateral surface of the catheter adapter housing, the second fluid flow conduit having a proximal end arranged on an upper surface of the catheter adapter housing and adjacent the reservoir and a distal end adjacent a bottom surface of the catheter adapter housing; and
a third fluid flow conduit in fluid communication with the second fluid flow conduit, the third fluid flow conduit extending along an outer surface of the catheter adapter housing parallel to a longitudinal axis defined by the catheter adapter lumen, the third fluid flow conduit having one or more openings configured to direct the adhesive to the skin of the patient.

7. The catheter adapter of claim 1, wherein the one or more fluid flow conduits are tubular.

8. The catheter adapter of claim 1, wherein the reservoir and/or the one or more fluid flow conduits are arranged on the catheter adapter housing by a press-fit or a friction fit.

9. The catheter adapter of claim 1, wherein the reservoir and/or the one or more fluid flow conduits are welded to the catheter adapter housing.

10. The catheter adapter of claim 9, wherein the reservoir and/or the one or more fluid flow conduits are tack welded or laser welded to the catheter adapter housing.

11. The catheter adapter of claim 8, wherein the catheter adapter housing comprises one or more grooves configured to receive at least a portion of the reservoir and/or the one or more fluid flow conduits.

12. The catheter adapter of claim 1, wherein the reservoir is spaced from an upper surface of the catheter adapter housing.

13. The catheter adapter of claim 1, wherein the reservoir is arranged on an upper surface of the housing.

14. A kit comprising:
a catheter adapter having a housing having a proximal end, a distal end, a lumen arranged between and in fluid communication with the distal end and the proximal end,
a catheter arranged at the distal end of the catheter adapter and in fluid communication with the lumen,
a reservoir,
an adhesive received within the reservoir, and
one or more fluid flow conduits in fluid communication with the reservoir and configured to direct the adhesive to the skin of a patient on which the catheter adapter is arranged; and
a packaging defining an interior, the catheter adapter received within the packaging interior.

15. The kit of claim 14, wherein the catheter adapter and/or the packaging are sterilized.
